# EUROPEAN PATENT APPLICATION

(11) **EP 4 570 265 A1**
(43) Date of publication of application: **18.06.2025**
(21) Application number: 23461694.4
(22) Date of filing: 15.12.2023
(51) Int. Cl.: A61K 39/085, A61K 39/09, A61P 31/04

(54) **VACCINE FOR USE IN PREVENTING INFLAMMATORY CONDITIONS IN COWS**

(71) Applicant: Zaklad Badawczo-Wdrozeniowy Osrodka Salmonella "Immunolab" Sp. z o.o., 80-822 Gdansk (PL)
(72) Inventor: LIEDER, Dorota, 83-050 Babi Dó (PL); G O NICKI, Krzysztof, 83-050 Babi Dó (PL)
(74) Representative: JD&P Patent Attorneys Joanna Dargiewicz & Partners

(57) **Abstract**

The subject matter of the present invention is a vaccine for use in the prevention and/or treatment of udder inflammation (mastitis) in cows, characterized in that it contains preparation a) and/or preparation b), wherein preparation a) contains as active substance S. *aureus* 4.2 LG₂ strain, inactivated with formaldehyde for intramammary administration, and preparation b) contains as active substance *S*. *uberis* 19MK and 7MO strains inactivated with formaldehyde for intramammary administration. The subject matter of the invention is also a method of manufacturing of such vaccine and the use thereof.

## Description

The present invention relates to a vaccine for use in the prevention of udder inflammations (mastitis) in cows.

Mastitis, udder inflammation, is a chronic, latent or acute inflammation of the mammary gland in animals; term also sometimes used in medicine for breast inflammation. A functional disorder of the mammary gland, proceeding under the influence of microorganisms that have entered the body. Clinical form (acute and chronic), subclinical (septic and aseptic). Udder inflammation causes a variety of qualitative changes in the milk, primarily reducing its technological suitability.

Each year, about 30% of Poland's dairy cattle population shows clinical mastitis symptoms and about 40% of cows show subclinical symptoms. Globally, this problem affects 20-45% of dairy cows, generating economic losses of €200/cow. According to the Central Statistical Office of Poland, the number of dairy cows in June 2019 was 2,233,000, which is about 1/3 of the cattle population in Poland. The methods of therapy and immunoprophylaxis of mastitis developed so far are not effective enough.

Mastitis is a multifactorial disease, both the host (cow), the pathogens and the environment are important. Pathogens most commonly isolated from cows include: *Staphylococcus aureus, Streptococcus agalactiae, Streptococcus uberis, Trueperella pyogenes, Escherichia coli.* During the course of the disease, the main changes in the milk (reducing its technological suitability) are: increased somatic cell count, concentration of chloride ions, level of the LDH enzyme, as well as the presence of secretory changes in the form of clots. Mastitis causes a decrease in a cow's milk yield by up to 17%. In addition to economic losses, all cases of the clinical form of mastitis cause pain and stress to the animal. The most important risk factors for mastitis incidence identified in the EFSA (2018) report are poor bedding hygiene and poorly designed, used or maintained milking equipment. Currently, treatment for clinical forms of the disease includes the administration of antibiotics and anti-inflammatory drugs. Such therapies show variable efficacy, due to increasing bacterial resistance to antibiotics, as well as untargeted treatment. Mastitis is a disease caused by several different pathogens, and therefore finding an effective antibiotic is problematic. In addition, there is an increase in drug resistance in dairy cattle, primarily to *S*. *aureus.* Too frequent administration of antibiotics can result in immune disorders, cause allergies and skin lesions, exacerbate or over-inhibit the inflammatory action of cytokines. The frequent use of antibiotics in the rearing of livestock from which food products are obtained is one of the sanitary and hygienic risks affecting public health.

Preparations immunizing against mastitis are also available on the market, but they are designed for administration by injection.

Patent document EP3417049 relates to an extract of *Streptococcus uberis* as an immunogenic agent. It also relates to a process for obtaining said agent, which comprises incubating a biofilm-producing *S. uberis* strain to obtain a biofilm and thermally treating the biofilm obtained. It also relates to a pharmaceutical composition comprising teichoic acids, preferably lipoteichoic acids, for use in the prevention and/or treatment of mastitis and/or infections caused by *Streptococcus sp.* or by biofilm-producing bacteria. This document also relates to a vaccine which comprises said immunogenic agent and to said vaccine and immunogenic agent for use in the prevention and/or treatment of infections caused by Streptococcus sp., particularly in the prevention and/or treatment of mastitis caused by *S. uberis.*

International patent application PCT/CL2015/050046 describes a polyvalent, highly immunogenic subunit vaccine against mastitis in mammals, made of a preparation comprising a mixture of cell wall and membrane fragments and small liposomes from the cell membrane of pathogens selected from *Staphylococcus aureus, Escherichia coli* and *Streptococcus uberis,* where said fragments of cell wall and membrane release immunostimulat intracellular elements such as membrane proteins, lipopolysaccharides, peptidoglycans, exopolysaccharides.

International patent application PCT/JP2022/034873 describes a vaccine for preventing infections by *Streptococcus spp.* that cause mastitis in livestock. Specifically, this document describes a vaccine that contains a nonencapsulated strain of *Streptococcus spp.* as an antigen, and in particular a vaccine for preventing mastitis caused by *Streptococcus uberis* infection.

Therefore, there is still a need in the field for new therapeutic and immunizing preparations that are not antibiotics.

So, the objective of the present invention was to develop new preparations for the prophylaxis and treatment of udder inflammation (mastitis) in cows, which could be an alternative to conventional chemotherapeutics and antibiotics.

This objective was achieved by the subject matter of the present invention, which is a vaccine for use in the prevention and/or treatment of udder inflammation (mastitis) in cows characterized in that it contains preparation a) and/or preparation b), wherein
preparation a) contains as an active substance *S. aureus* 4.2 LG₂ strain, inactivated with formaldehyde, for intramammary administration, with the following characteristics:

| Feature/strain | 4.2 LG₂ |
|---|---|
| Identification to species | *nuc* |
| Toxicity genes | - |
| Genes encoding surface proteins | *IsdC, EsxA, IsdA, SdrD, IsdB, Bap,* and *SdrC* |
| Antibiotic resistance | to most antibiotics tested, including cloxacillin, cefoperazone, cefquinome, tetracycline, neomycin, kanamycin, and gentamicin |
| Sensitivity | amoxicillin plus clavulanic acid, penicillin, cephalexin, streptomycin, and bacitracin |

biochemical diagnostics (ErbaScan) for *S. aureus* 4.2 LG strain

| URE | ARG | ORN | BGA | GLR | BGL | PHS | ESL | NAG | GAL | SUC | TRE | MAN | MLT | XYL | MNS |
|---|---|---|---|---|---|---|---|---|---|---|---|---|---|---|---|
| + | + | - | - | - | + | + | + | + | + | + | + | + | + | - | + |

| LAC | SOR | RIB | FRU | CEL | ARA | RAF | XOL |
|---|---|---|---|---|---|---|---|
| + | - | + | + | + | - | - | - |

preparation b) contains as an active ingredient *S. uberis* 19MK and 7MO strains, inactivated with formaldehyde, for intramammary administration, with the following characteristics:

| Feature/strain | 19MK |
|---|---|
| Identification to species | *165 rRNA* |
| Toxicity genes | *cfu* |
| Genes encoding surface proteins | *gapC* |
| Antibiotic resistance | to all antibiotics tested |

biochemical diagnostics (ErbaScan) for 19MK

| HIP | PHS | LAP | GLR | AGA | ESL | ARG | URE | MAN | SOR | TRE | LAC | RAF | INU | MLB | RIB |
|---|---|---|---|---|---|---|---|---|---|---|---|---|---|---|---|
| + | - | + | - | - | + | + | - | + | + | + | + | - | - | - | + |

| Feature/strain | 7MO |
|---|---|
| Identification to species | *165 rRNA* |
| Toxicity genes | *sua* |
| Genes encoding surface proteins | *skc, pauA, gapC, oppF, hasA*, *hasB, hasC* |
| Antibiotic resistance | resistant to most antibiotics tested |
| Sensitivity | shows sensitivity or medium sensitivity to penicillin, cephalexin, erythromycin, and bacitracin |

biochemical diagnostics (ErbaScan) for 7MO

| HIP | PHS | LAP | GLR | AGA | ESL | ARG | URE | MAN | SOR | TRE | LAC | RAF | INU | MLB | RIB |
|---|---|---|---|---|---|---|---|---|---|---|---|---|---|---|---|
| + | - | + | + | - | + | + | - | + | + | + | + | - | - | - | + |

Preferably, preparation a) contains 0.4×10⁸ CFU/mL (2×10⁹ CFU in 5 mL) of a formaldehyde-inactivated *S. aureus* strain.

Preferably, preparation b) contains 0.2×10⁸ CFU/mL of *S. uberis* 19MK strain and 0.2×10⁸ CFU/mL of *S. uberis* 7MO strain inactivated with formaldehyde.

Preferably, the vaccine additionally contains preparation c) containing further inactivated bacterial strains, preferably *Escherichia coli, Klebsiella pneumoniae* and *Klebsiella oxytoca* and *Streptococcus dysgalactiae.*

Preferably, the vaccine as additives in all preparations contains saline, hydrogel (10% glycerol in water, 1% hydroxyethylcellulose powder with a viscosity of 1500 to 2500 cps), formaldehyde.

Preferably, the vaccine is administered according to the following schedule:

| **Administration** | **Age of the animal** | **Composition of the intramammary dose** |
|---|---|---|
| I dose to each teat in volume of 5 mL | heifers min. 20 months old, about 4 weeks before calving | 0.4×10⁸ CFU/mL (2×10⁹ CFU in 5 mL) of inactivated *S. aureus* 4.2 LG₂ strain bacteria or inactivated *S. uberis* bacteria, containing 0.2×10⁸ CFU/mL of 19MK strain and 7MO strain (mixed 1:1, total of 0.4×10⁸ CFU/mL → 2×10⁹ CFU in 5 mL) and suspended in 1% hydrogel. |
| II dose to each teat in volume of 5 mL | heifers min. 20 months old, about 2 weeks before calving | 0.4×10⁸ CFU/mL (2×10⁹ CFU in 5 mL) of inactivated *S. aureus* 4.2 LG₂ strain bacteria or inactivated *S. uberis* bacteria, containing 0.2×10⁸ CFU/mL of 19MK strain and 7MO strain (mixed 1:1, total of 0.4×10⁸ CFU/mL → 2×10⁹ CFU in 5 mL) and suspended in 1% hydrogel. |

or

| **Administration** | **Age of the animal** | **Composition of the intramammary dose** |
|---|---|---|
| I dose to each teat in volume of 5 mL | heifers min. 20 months old, about 4 weeks before calving | 0.4×10⁸ CFU/mL (2×10⁹ CFU in 5 mL) of inactivated *S. aureus* 4.2 LG₂ strain bacteria and suspended in 1% hydrogel. |
| II dose to each teat in volume of 5 mL | heifers min. 20 months old, about 2 weeks before calving | 0.4×10⁸ CFU/mL (2×10⁹ CFU in 5 mL) of inactivated *S. aureus* 4.2 LG₂ strain bacteria and suspended in 1% hydrogel. |
| III dose immediately after calving, to each teat in volume of 5 mL | heifers min. 20 months old, on the day of parturition | 0.4×10⁸ CFU/mL (2×10⁹ CFU in 5 mL) of inactivated *S. aureus* 4.2 LG₂ strain bacteria and suspended in 1% hydrogel. |

Also a subject matter of the invention is a method for obtaining a vaccine as defined above, comprising steps in which:
- a culture containing at least one strain selected from such as *Staphylococcus aureus, Streptococcus uberis, Escherichia coli, Klebsiella pneumoniae* and *Klebsiella oxytoca* and *Streptococcus dysgalactiae* is inactivated with 10% formalin to obtain a concentration of 0.1% and incubated at 37 °C with stirring without air for at least 3h;
- the culture is then transferred to a sterile vessel and inactivated while tightly sealed overnight under the same conditions;
- the inactivated bacteria are centrifuged and the pellet is suspended in 0.85% NaCl solution with 0.02% formalin;
- a hydrogel containing 10% glycerol in water is prepared, heated to at least 95 °C, then after cooling to about 80-90 °C while stirring, hydroxyethylcellulose with a viscosity of 1500 to 2500 cps in powder form is added to a 1% concentration and the hydrogel is stirred until completely cooled;
- the suspension of inactivated bacterial strains obtained above is added to the resulting hydrogel and stirred.

The invention also relates to a use of the vaccine defined above for the manufacture of an immunizing and/or therapeutic preparation against udder inflammation (mastitis) in cows.

The authors of the present invention have developed immunological preparations for the prophylaxis of udder inflammation (mastitis) in cows, which can be an alternative to conventional chemotherapeutics and antibiotics. A vaccine has been developed against the pathogens that cause the most severe symptoms of the disease, which is administered in hydrogel form as an intramammary infusion.

The vaccine according to the present invention contains a) and/or b)
*a) Staphylococcus aureus,* containing 0.4×10⁸ CFU/mL (2×10⁹ CFU in 5 mL) of 4.2 LG₂ strain
b) *Streptococcus uberis,* containing 0.2×10⁸ CFU/mL of 19MK strain and 0.2×10⁸ CFU/mL of 7MO strain (total of 0.4×10⁸ CFU/mL → 2×10⁹ CFU in 5 mL).

Characteristics of the selected strains:

### 1. Staphylococcus aureus

### Immunolab Collection Number: 4.2 LG₂

Origin of the strain: Strain isolated from milk from a cow with mastitis symptoms. The sample was collected in March 2021 from the farm of Mariusz Kijańczyk in the village of G 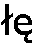 bock.

Storage and transportation conditions: The milk sample was collected into a sterile container and transported under refrigeration to Immunolab company.
Growth media: Soybean casein broth, Columbia Agar with 5% sheep blood
Differentiation media: Columbia CNA Agar, Chromogenic S. aureus Agar
Bacterial growth: Yellow, shiny, beta-hemolytic colonies on Columbia CNA medium, growth at 37 °C; pink colonies on Chromogenic S. aureus medium, growth at 37 °C, after culturing in liquid medium after inactivation easy to pellet by centrifugation, pellet intense yellow or orange, growth very abundant
Culture media: Enriched or soybean casein broths
Biochemical features:

| **Name of the method:** STAPHYtest 24 | | |
|---|---|---|
| **Identified strain:** *Staphylococcus aureus* subsp. *aureus* | | |
| **Evaluation:** Good identification (ID: 93.30, T-index: 0.618) | | |
| **Test** | **Abbreviation** | **Reaction** |
| Urease | URE | + |
| Arginine | ARG | + |
| Ornithine | ORN | - |
| β-Galactosidase | bGA | - |
| β-Glucuronidase | GLR | - |
| β-Glucosidase | bGL | + |
| Phosphatase | PHS | + |
| Esculin | ESL | - |
| N-acetyl-0-D-glucosamine | NAG | + |
| Galactose | GAL | + |
| Sucrose | SUC | + |
| Trehalose | TRE | + |
| Mannitol | MAN | + |
| Maltose | MLT | + |
| Xylose | XYL | - |
| Mannose | MNS | + |
| Lactose | LAC | - |
| Sorbitol | SOR | - |
| Ribose | RIB | - (visual evaluation) |
| Fructose | FRU | + |
| Cellobiose | CEL | - |
| Arabinose | ARA | - (visual evaluation) |
| Raffinose | RAF | - |
| Xylitol | XOL | - |

Catalase test: positive (gas bubble formation)
Serological features:
The strain was tested with the Staph Latex Kit test (Pro-Lab): positive (aggregate formation)
Antibiotic resistance:

| **Abbreviation and concentration of the antibiotic** | **Name of the antibiotic** |
|---|---|
| AMC 30 | Amoxicillin plus clavulanic acid |
| P1 | Penicillin |
| CX 5 | Cloxacillin |
| CL 30 | Cephalexin |
| CEP 30 | Cefoperazone |
| CFQ 30 | Cefquinome |
| TE 30 | Tetracycline |
| N 30 | Neomycin |
| S 300 | Streptomycin |
| K 30 | Kanamycin |
| CN 10 | Gentamicin |
| B 10 | Bacitracin |

| **Strain tested** | **Antibiotic** | | | | | | | | | | | |
|---|---|---|---|---|---|---|---|---|---|---|---|---|
| | **AMC 30** | **P1** | **CX 5** | **CL 30** | **CEP 30** | **CFQ 30** | **TE 30** | **N 30** | **S 300** | **K 30** | **CN 10** | **B 10** |
| ***S*. *aureus* 4.2** LG₂ | S | S | MS | S | MS | MS | MS | MS | S | MS | MS | S |

S: sensitive
MS: medium sensitive
R: resistant

### Genetic features:

The strain expresses a wide range of virulence factors that include surface proteins covalently attached to the cell wall and proteins secreted during infection. These proteins include: **IsdC** - iron-regulated surface determinant protein C, **EsxA** - ESAT-6 secretory system extracellular protein, **IsdA** - surface adhesin, involved in iron sequestration, **IsdB** - adhesin, **Bap** - surface protein involved in biofilm formation, **SdrD, SrdC** - a subfamily of cell wall-anchored proteins called MSCRAMMs (microbial surface component recognizing adhesive matrix molecules) mediate adhesion to host tissues.

### 2. Streptococcus uberis

### Immunolab Collection Number: 7MO

Origin of the strain: Strain isolated from milk from a cow with mastitis symptoms. The sample was collected in March 2021 from the farm of Marek Wroński in the village of Szczepkowo-Giewarty, Janowo district, marked 4/2021 MO, 143 PT. Received from the Polish Academy of Sciences (PAS) Olsztyn.

Storage and transportation conditions: The milk sample was collected into a sterile container and frozen. After collecting more samples, they were transported under refrigeration to Immunolab company.
Growth media: Soybean casein broth, Columbia Agar with 5% sheep blood
Differentiation media: Columbia CNA Agar, Chromogenic Strepto B Agar
Bacterial growth: Blue-gray small colonies on Columbia CNA Agar, growth at 37 °C; blue colonies on Chromogenic Strepto B Agar, growth at 37 °C, growth not very abundant in liquid medium, white pellet after centrifugation
Culture media: Enriched or soybean casein broths
Biochemical features:

| **Name of the method:** STREPTOtest 16 | | |
|---|---|---|
| **Identified strain:** Streptococcus *uberis* | | |
| **Evaluation:** Very good identification (ID: 98.60, T-index: 1.000) | | |
| **Test** | **Abbreviation** | **Reaction** |
| Hippurate | HIP | + |
| Phosphatase | PHS | - |
| Leucine aminopeptidase | LAP | + |
| β-Glucuronidase | GLR | + |
| α-Galactosidase | aGA | - |
| Esculin | ESL | + |
| Arginine | ARG | + |
| Urease | URE | - |
| Mannitol | MAN | + |
| Sorbitol | SOR | + |
| Trehalose | TRE | + |
| Lactose | LAC | + |
| Raffinose | RAF | - |
| Inulin | INU | - |
| Mellibiose | MLB | - |
| Ribose | RIB | + |

Catalase test: negative (no gas bubbles)
Serological features:
The strain was tested with the Streptococcal Grouping Latex Kit (Pro-Lab) for typing streptococci into groups A, B, C, D, F, G according to Lancefield-negative result for all latexes (no agglutination).
Antibiotic resistance:

| **Abbreviation and concentration of the antibiotic** | **Name of the antibiotic** |
|---|---|
| AX 25 | Amoxicillin |
| P 1 | Penicillin |
| AM 10 | Ampicillin |
| CX 5 | Cloxacillin |
| CL 30 | Cephalexin |
| CEP 30 | Cefoperazone |
| CFQ 30 | Cefquinome |
| E 15 | Erythromycin |
| TE 30 | Tetracycline |
| N 30 | Neomycin |
| L 15 | Lincomycin |
| B 10 | Bacitracin |

| **Strain tested** | **Antibiotic** | | | | | | | | | | | |
|---|---|---|---|---|---|---|---|---|---|---|---|---|
| | **AX 25** | **P 1** | **AM 10** | **CX 5** | **CL 30** | **CEP 30** | **CFQ 30** | **E 15** | **TE 30** | **N 30** | **L 15** | **B 10** |
| ***S*. *uberis* 7MO** | R | S | R | R | S | R | R | MS | R | R | R | S |

S: sensitive
MS: medium sensitive
R: resistant

### Genetic features:

The strain has genes: sua - gene encoding adhesin protein, responsible for adhesion and invasion of epithelial cells, pauA/skc - plasminogen activators, responsible for the colonization, *gapC* - encodes surface dehydrogenase protein responsible for the colonization, *hasA*, *hasB, hasC* - hyaluronic acid capsules, responsible for resistance to phagocytosis, *oppF* - gene encoding an oligopeptide permease co-forming oligopeptide transport system.

### 3. Streptococcus uberis

### Immunolab Collection Number: 19MK

Origin of the strain: Strain isolated from milk from a cow with mastitis symptoms. The sample was collected in February 2021 from a farm near Kartury. The sample was collected and donated by vet. Przemys 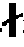 aw Warmowski.

Storage and transportation conditions: The milk sample was collected into a sterile container and transported under refrigeration to Immunolab company. The sample was stored in a freezer.
Growth media: Soybean casein broth, Columbia Agar with 5% sheep blood
Differentiation media: Columbia CNA Agar, Chromogenic Strepto B Agar
Bacterial growth: Blue-gray small colonies on Columbia CNA Agar, growth at 37 °C; blue colonies on Chromogenic Strepto B Agar, growth at 37 °C, growth not very abundant in liquid medium, white pellet after centrifugation
Culture media: Enriched or soybean casein broths
Biochemical features:

| **Name of the method:** STREPTOtest 16 | | |
|---|---|---|
| **Identified strain:** Streptococcus *uberis* | | |
| **Evaluation:** Very good identification (ID: 98.60, T-index: 1.000) | | |
| **Test** | **Abbreviation** | **Reaction** |
| Hippurate | HIP | + |
| Phosphatase | PHS | - |
| Leucine aminopeptidase | LAP | + |
| β-Glucuronidase | GLR | + |
| α-Galactosidase | aGA | - |
| Esculin | ESL | + |
| Arginine | ARG | + |
| Urease | URE | - |
| Mannitol | MAN | + |
| Sorbitol | SOR | + |
| Trehalose | TRE | + |
| Lactose | LAC | + |
| Raffinose | RAF | - |
| Inulin | INU | - |
| Mellibiose | MLB | - |
| Ribose | RIB | + |

Catalase test: negative (no gas bubbles)
Serological features:
The strain was tested with Streptococcal Grouping Latex Kit (Pro-Lab) for typing streptococci into groups A, B, C, D, F, G according to Lancefield-negative result for all latexes (no agglutination).
Antibiotic resistance:

| **Abbreviation and concentration of the antibiotic** | | | **Name of the antibiotic** | | | | | | | | | |
|---|---|---|---|---|---|---|---|---|---|---|---|---|
| AX 25 | | | Amoxicillin | | | | | | | | | |
| P 1 | | | Penicillin | | | | | | | | | |
| AM 10 | | | Ampicillin | | | | | | | | | |
| CX 5 | | | Cloxacillin | | | | | | | | | |
| CL 30 | | | Cephalexin | | | | | | | | | |
| CEP 30 | | | Cefoperazone | | | | | | | | | |
| CFQ 30 | | | Cefquinome | | | | | | | | | |
| E 15 | | | Erythromycin | | | | | | | | | |
| TE 30 | | | Tetracycline | | | | | | | | | |
| N 30 | | | Neomycin | | | | | | | | | |
| L 15 | | | Lincomycin | | | | | | | | | |
| B 10 | | | Bacitracin | | | | | | | | | |

| **Strain tested** | **Antibiotic** | | | | | | | | | | | |
|---|---|---|---|---|---|---|---|---|---|---|---|---|
| | **AX 25** | **P 1** | **AM 10** | **CX 5** | **CL 30** | **CEP 30** | **CFQ 30** | **E 15** | **TE 30** | **N 30** | **L 15** | **B 10** |
| ***S*. *uberis* 19MK** | R | R | R | R | R | R | R | R | R | R | R | R |

S: sensitive
MS: medium sensitive
R: resistant

### Genetic features:

The strain has the *cfu* gene, which encodes the cAMP factor, responsible for creating pores in the host cell membrane, allowing entry into the cell, and the *gapC* gene, which encodes the surface dehydrogenase protein, responsible for colonization.

**Target animal species:** The products are designed for cows of all ages, including heifers (mainly up to 2 years old).

### Therapeutic indication for specific target animal species:

Vaccine: Active immunization of healthy cows and heifers (including in-calf cows and heifers immediately after calving) to reduce the incidence of clinical and subclinical mastitis caused by *Streptococcus uberis, Staphylococcus aureus* or other bacteria underlying the pathogenesis of the udder inflammation, prevent or reduce the severity of infection, reduce the somatic cell count (SCC) in quarters infected with the above-mentioned pathogens and reduce milk production losses caused by udder infection.

Laboratory and field studies have demonstrated the protective effect of the vaccine against pathogens that cause mastitis, as well as the therapeutic effect of the preparation, which inactivates pathogens present in the cow's udder and mobilizes the cow's immune system to fight the developing infection.

### DETAILED DESCRIPTION OF THE INVENTION

The vaccine according to the present invention contains preparation a) and/or preparation b) and/or preparation c)
Preparation a):
**Qualitative and quantitative composition**
Active substance: *S*. *aureus* 4.2 LG₂ strain containing 0.4×10⁸ CFU/mL (2×10⁹ CFU in 5 mL) of the strain, inactivated with formaldehyde
**Administration route**
Intramammary
**Strain characteristics**
Sensitivity to the following antibiotics was determined using the disk diffusion method: amoxicillin plus clavulanic acid, penicillin, cloxacillin, cephalexin, cefoperazone, cefquinome, tetracycline, neomycin, streptomycin, kanamycin, gentamycin, bacitracin.

Genetic analysis of the strain, which was isolated from the milk of cows suffering from mastitis, was carried out. Pathogenicity and toxicity genes of the strains were searched for in order to select the best candidates for a vaccine against the disease. For each strain, the species affiliation and virulence, pathogenicity and toxicity genes, responsible, according to the literature, for udder inflammation in cows were examined. In *Staphylococcus aureus,* the species affiliation and toxicity genes were examined, *i.e. sea, seb,* sec, sed, see, tst, eta, etb and genes encoding surface proteins enabling adhesion to host cells, as well as those responsible, *i.a*., for biofilm formation: *IsdC, EsxA, IsdA, SdrD, IsdB, Bap, SdrC.*

Biochemical identification of bacteria isolated from milk samples from cows with mastitis symptoms was carried out.

### Analytical results for S. aureus 4.2 LG₂ strain

| **Feature/strain** | **4.2 LG₂** |
|---|---|
| **Identification to species** | nuc |
| **Toxicity genes** | - |
| **Genes encoding surface proteins** | *IsdC, EsxA, IsdA, SdrD, IsdB, Bap,* and *SdrC* |
| **Antibiotic resistance** | to most antibiotics tested, including cloxacillin, cefoperazone, cefquinome, tetracycline, neomycin, kanamycin, and gentamicin |
| **Sensitivity** | amoxicillin plus clavulanic acid, penicillin, cephalexin, streptomycin, and bacitracin |

### Biochemical diagnostics (ErbaScan) for S. aureus 4.2 LG strain

| URE | ARG | ORN | BGA | GLR | BGL | PHS | ESL | NAG | GAL | SUC | TRE | MAN | MLT | XYL | MNS |
|---|---|---|---|---|---|---|---|---|---|---|---|---|---|---|---|
| + | + | - | - | - | + | + | + | + | + | + | + | + | + | - | + |

| LAC | SOR | RIB | FRU | CEL | ARA | RAF | XOL |
|---|---|---|---|---|---|---|---|
| + | - | + | + | + | - | - | - |

Preparation b):
**Qualitative and quantitative composition**
Active ingredients: *S*. *uberis* 19MK strain containing 0.2×10⁸ CFU/mL and 7MO strain containing 0.2×10⁸ CFU/mL (total of 0.4×10⁸ CFU/mL → 2×10⁹ CFU in 5 mL) inactivated with formaldehyde
**Administration route**
Intramammary
**Strain characteristics**
Sensitivity to the following antibiotics was determined using the disk diffusion method: i.e. amoxicillin, penicillin, ampicillin, cloxacillin, cephalexin, cefoperazone, cefquinome, erythromycin, tetracycline, neomycin, lincomycin, and bacitracin.

Genetic analysis of strains isolated from the milk of cows suffering from mastitis was carried out. Pathogenicity and toxicity genes of the strains were searched for in order to select the best candidates for a vaccine against the disease. For each strain, species affiliation and virulence, pathogenicity and toxicity genes, responsible, according to the literature, for udder inflammation in cows were examined. In Streptococcus *uberis* the species affiliation was examined, *cfu* - cAMP factor gene, *sua* - adhesion protein gene, virulence genes encoding, *i.a*., lactoferrin binding proteins, factors preventing phagocytosis, factors responsible for colonization - *skc, pauA, gapC, oppF, Ibp, hasA, hasB, hasC.*

| **Feature/strain** | **19MK** |
|---|---|
| **Identification to species** | *165 rRNA* |
| **Toxicity genes** | *cfu* |
| **Genes encoding surface proteins** | *gapC* |
| **Antibiotic resistance** | to all antibiotics tested |

### Biochemical diagnostics (ErbaScan) for 19MK

| HIP | PHS | LAP | GLR | AGA | ESL | ARG | URE | MAN | SOR | TRE | LAC | RAF | INU | MLB | RIB |
|---|---|---|---|---|---|---|---|---|---|---|---|---|---|---|---|
| + | - | + | - | - | + | + | - | + | + | + | + | - | - | - | + |

| **Feature/strain** | **7MO** |
|---|---|
| **Identification to species** | *165 rRNA* |
| **Toxicity genes** | *sua* |
| **Genes encoding surface proteins** | *skc*, *pauA, gapC, oppF, hasA, hasB, hasC* |
| **Antibiotic resistance** | resistant to most antibiotics tested |
| **Sensitivity** | shows sensitivity or medium sensitivity to penicillin, cephalexin, erythromycin, and bacitracin |

### Biochemical diagnostics (ErbaScan) for 7MO

| HIP | PHS | LAP | GLR | AGA | ESL | ARG | URE | MAN | SOR | TRE | LAC | RAF | INU | MLB | RIB |
|---|---|---|---|---|---|---|---|---|---|---|---|---|---|---|---|
| + | - | + | + | - | + | + | - | + | + | + | + | - | - | - | + |

Preparation c):
In addition to preparations a) and b), the vaccine according to the present invention may contain further inactivated bacterial strains, in particular *Escherichia coli, Klebsiella pneumoniae* and *Klebsiella oxytoca,* and *Streptococcus dysgalactiae.*

Saline, hydrogel (10% glycerol in water, 1% hydroxyethylcellulose powder with medium viscosity, molecular weight of 170,000, viscosity of 1500-2500 cps), formaldehyde are used as additives in all preparations.

### Vaccine administration schedule

| **Administration** | **Age of the animal** | **Composition of the intramammary dose** |
|---|---|---|
| I dose to each teat in volume of 5 mL | heifers min. 20 months old, about 4 weeks before calving | 0.4×10⁸ CFU/mL (2×10⁹ CFU in 5 mL) of inactivated *S*. *aureus* 4.2 LG₂ strain bacteria or inactivated *S. uberis* bacteria, containing 0.2×10⁸ CFU/mL of 19MK strain and 7MO strain (mixed 1:1, total of 0.4×10⁸ CFU/mL → 2×10⁹ CFU in 5 mL) and suspended in 1% hydrogel. |
| II dose to each teat in volume of 5 mL | heifers min. 20 months old, about 2 weeks before calving | 0.4×10⁸ CFU/mL (2×10⁹ CFU in 5 mL) of inactivated *S. aureus* 4.2 LG₂ strain bacteria or inactivated *S. uberis* bacteria, containing 0.2×10⁸ CFU/mL of 19MK strain and 7MO strain (mixed 1:1, total of 0.4×10⁸ CFU/mL → 2×10⁹ CFU in 5 mL) and suspended in 1% hydrogel. |

OR

| **Administration** | **Age of the animal** | **Composition of the intramammary dose** |
|---|---|---|
| I dose to each teat in volume of 5 mL | heifers min. 20 months old, about 4 weeks before calving | 0.4×10⁸ CFU/mL (2×10⁹ CFU in 5 mL) of inactivated *S. aureus* 4.2 LG₂ strain bacteria and suspended in 1% hydrogel. |
| II dose to each teat in volume of 5 mL | heifers min. 20 months old, about 2 weeks before calving | 0.4×10⁸ CFU/mL (2×10⁹ CFU in 5 mL) of inactivated *S. aureus* 4.2 LG₂ strain bacteria and suspended in 1% hydrogel. |
| III dose immediately after calving, to each teat in volume of 5 mL | heifers min. 20 months old, on the day of parturition | 0.4×10⁸ CFU/mL (2×10⁹ CFU in 5 mL) of inactivated S. *aureus* 4.2 LG₂ strain bacteria and suspended in 1% hydrogel. |

### EXAMPLES

### Example 1

### Genetic identification of strains isolated from milk of cows suffering from mastitis

The objective of the study was to perform a genetic analysis of strains isolated from milk of cows suffering from mastitis. Pathogenicity and toxicity genes of the strains were searched for in order to select the best candidates for a vaccine against the disease. Genetic analysis was carried out for mastitis-causing strains most commonly present in field samples, *i.e. Escherichia coli, Klebsiella, Streptococcus uberis, Staphylococcus aureus, Streptococcus agalactiae, Streptococcus dysgalactiae.*

The first step was the isolation of bacterial DNA. Commercially available DNA isolation kits were used for this purpose. For *S. aureus,* the Genomic Mini AX Staphylococcus kit from A&A Biotechnology was used, for Streptococcus and *Trueperella* a kit dedicated to Gram-positive bacteria was used: Gram Plus & Yeast Genomic DNA Purification Kit from EURx, while for *E coli* and *Klebsiella,* the Bacteria & Yeast Genomic DNA Purification Kit was used. For each strain, species affiliation and virulence, pathogenicity and toxicity genes responsible for udder inflammation in cows were tested by PCR.

Composition of the reaction mixture:
Water: 4.1 µL
PCR Mix (A&A Biotechnology): 5 µL
Primer F: 0.2 µl
Primer R: 0.2 µl
Template: 0.5 µl

Samples were applied to an agarose gel (the percentage of the gel depended on the size of the product) and separated for 30 min, 100 V, and then visualized in a transilluminator.

A total of 235 samples were tested, of which pathogenic ones turned out to be:
42 *S. aureus* strains,
12 *S. dysgalactiae* strains,
2 *S*. *agalactiae* strains,
45 *S. uberis* strains,
16 *E*. *coli* strains,
10 *Klebsiella spp*. strains,
1 *Trueperella* strain.

For each strain, species affiliation and virulence, pathogenicity and toxicity genes, responsible, according to the literature, for udder inflammation in cows, were studied.
• *Staphylococcus aureus:* species affiliation was examined and
- *sea, seb, sec, sed, see, tst, eta, etb* toxicity genes
- *IsdC, EsxA, IsdA, SdrD, IsdB, Bap, SdrC* genes encoding surface proteins that enable adhesion to host cells and are also responsible *i.a.* for biofilm formation,

| gene | % |
|---|---|
| *IsdC* | 100 |
| *EsxA* | 100 |
| *IsdA* | 100 |
| *SdrD* | 59.5 |
| *IsdB* | 95.2 |
| *Bap* | 45.2 |
| *SdrC* | 95.2 |
| *sea* | 7.1 |
| *seb* | 4.8 |
| *sec* | 7.1 |
| *sed* | 7.1 |
| *see* | 0 |
| *tst* | 2.4 |
| *eta* | 0 |
| *etb* | 0 |

### 35% of strains tested have all pathogenicity genes

• *Streptococcus agalactiae* species affiliation and
- *rib, Alpha C, Alpha* 2/3, *Alpha* 4, *Epsilon* genes encoding surface proteins-serotype identification

| gene | % |
|---|---|
| *rib* | 0 |
| *Alpha C* | 100 |
| *Alpha 2*/*3* | 0 |
| *Alpha 4* | 0 |
| *Epsilon* | 0 |

• *Klebsiella spp*. species affiliation and
- *K*1, K2, *rmpA, uge, kfu, magA, aerobactin* virulence genes

| gene | % |
|---|---|
| *K1* | 20 |
| *K2* | 0 |
| *rmpA* | 0 |
| *uge* | 80 |
| *kfu* | 80 |
| *magA* | 0 |
| *aerobactin* | 5 |

• *Streptococcus uberis* species affiliation and
- *cfu*: cAMP factor gene
- *sua*: adhesion protein gene
- *skc, pauA, gapC, oppF, Ibp, hasA, hasB, hasC* virulence genes, encoding i.a. lactoferrin binding proteins, factors that prevent phagocytosis, factors responsible for colonization

| gene | % |
|---|---|
| *cfu* | 4.4 |
| *skc* | 77.8 |
| *sua* | 82.2 |
| *pauA* | 73.3 |
| *gapC* | 88.9 |
| *oppF* | 77.8 |
| *lbp* | 0 |
| *hasA* | 75.6 |
| *hasB* | 75.6 |
| *hasC* | 48.9 |

• *Streptococcus dysgalactiae* species affiliation and
- *napr, eno* virulence genes

| gene | % |
|---|---|
| *napr* | 100 |
| *eno* | 75 |

### 80% of strains tested have all pathogenicity genes tested

• *Escherichia coli* species affiliation and
- *bfpEP, eae, esp, aggR, elt, ipaH, stx1*, *stx2* genes encoding pathogenicity factors, i.a. adhesins, intimins, Shiga toxin

| gene | % |
|---|---|
| *bfpEP* | 0 |
| *eae* | 375 |
| *esp* | 12.5 |
| *aggR* | 6.3 |
| *elt* | 12.5 |
| *ipaH* | 6.25 |
| *stx1* | 43.8 |
| *stx2* | 0 |

### RESULTS

Based on the above tests, candidate strains for the cow vaccine were selected. The strains were selected so that they collectively contained as many virulence features described in the literature as associated with mastitis development as possible. The following strains were selected:

### E coli:

- 16L1: *ipaH*
- PAN 4/1: elt
- 4MO1: *stx1*
- 13MO2: *eae, sxt1*

### Klebsiella spp.:

- 42MO1 (*K*. *pneumoniae*): *K1, aerobactin*
- 7L2 (*K oxytoca*): *uge, kfu*

### Staphylococcus aureus:

- 4.2 LG₂: *nuc, IsdC, EsxA, IsdA, SdrD, IsdB, Bap, SdrC*

### Streptococcus uberis:

- 19MK: *cfu, gapC*
- 7MO: *skc, sua, pauA, gapC, oppF, hasA, hasB, hasC*

### Streptococcus dysgalactiae:

- 2MO₂: *napr, eno*

### Example 2

### Preparation of hydrogel with antigen addition

The objective of the experiments was to prepare hydrogel of different densities and to add antigen to the selected versions. A literature recipe for hydrogel was used as a starting point: 5% glycerol, 2.5% HEC (hydroxyethylcellulose). In addition, versions with 1.5% and 3.5% HEC were prepared. An ELISA assay was also conducted to determine whether it would be a suitable assay to verify the amount of antigen in the hydrogel to QC of the product.

Three versions of the hydrogel were prepared, each of 50 mL: 1.5%, 2.5% and 3.5% according to the recipe: a mixture of water and 5% glycerol was heated with stirring to at least 95° C, then HEC was gradually added and the mixture was cooled with constant stirring.

CONCLUSIONS: At room temperature, the 1.5% gel is quite liquid, the 2.5% one is not very liquid and the 3.5% one is practically solid.

The hydrogels were stored in a refrigerator until further analysis.

The 3.5% gel was rejected due to its too dense structure, and the 1.5% and 2% gels were further analyzed.

From each gel, 10 mL was taken for storage, and antigen was added to the remaining 40 mL. Inactivated S. *aureus* 4.2 LG₂ strain antigen was prepared and the amount needed to be added to the 40 mL gel was calculated (4 doses of 10 mL each). A dose of 5×10⁹ was assumed (5×10⁸ CFU/mL in a 10 mL dose).

An appropriate amount of antigen was added to 40 mL of hydrogels and the whole was stirred with a stirring rod.

CONCLUSIONS: The 1.5% gel stirred easily, few bubbles formed, with the 2.5% gel many air bubbles formed, the gel was very thick after storage in the refrigerator.

### Example 3

### 3.1 Formulation of preparations for in-calf heifers immunization (vaccine)

The objective of the experiments was to prepare doses of the preparation for administration to animals, the in-calf heifers, for immunization. It was planned to administer 2 doses of the preparation about 4 and 2 weeks before calving. The preparation was to be administered to all four quarters of the udder (4 teats) in a volume of 5 mL, each dose to contain 2×10⁹ CFU of inactivated bacteria. Two preparations will be tested: (i) against *S. aureus,* containing 0.4×10⁸ CFU/mL (2×10⁹ CFU in 5 mL) of 4.2 LG₂ strain, (ii) against S. uberis, containing 0.2×10⁸ CFU/mL of 19MK strain and 0.2×10⁸ CFU/mL of 7MO strain (total of 0.4×10⁸ CFU/mL → 2×10⁹ CFU in 5 mL).

Three bottles of 500 mL hydrogel base (10% glycerol in water) were prepared and placed in an autoclave. A magnetic stir bar was placed in each bottle before autoclaving.

After removal from the autoclave, the hot hydrogel was placed on a magnetic stirrer and, after cooling to about 80-90 °C while stirring all the time, hydroxyethylcellulose powder (medium viscosity, molecular weight of 170,000, viscosity of 1500-2500 cps) was added to obtain 1% concentration. The hydrogel was left on the stirrer to cool completely.

The contents of one bottle were aliquoted as a control preparation, hydrogel only, 55-60 g each into sterile injection bottles (6 pcs → 330-360 g total).

*S. uberis* or *S. aureus* antigens were added to the other two bottles in an amount equivalent to 100 doses (500 mL corresponds to 100 doses of 5 mL each) that is, 2×10¹¹ CFU.

The gel with antigens was also aliquoted, each into 6 injection bottles of 55-60 g.

Sterility testing cultures were run from each of the last used bottles, 0.5 mL each, and the sterility of the preparations was read: STERILE.

### 3.2 Formulation of preparations for cows immunization (vaccine)

The objective of the experiments is to prepare the 3rd dose of the preparation for administration to animals for immunization immediately after parturition. Administration of the first 2 doses of the preparation, about 4 and 2 weeks before calving and the 3rd dose on the day of parturition after colostrum collection. The preparation is to be administered to all four quarters of the udder (4 teats) in a volume of 5 mL, each dose will contain 2×10⁹ CFU of inactivated bacteria. Two preparations will be tested: (i) against *S. aureus,* containing 0.4×10⁸ CFU/mL (2×10⁹ CFU in 5 mL) of 4.2 LG₂ strain, (ii) against *S. uberis*, containing 0.2×10⁸ CFU/mL of 19MK strain and 0.2×10⁸ CFU/mL of 7MO strain (total of 0.4×10⁸ CFU/mL → 2×10⁹ CFU in 5 mL).

A bottle with 600 mL of hydrogel base (10% glycerol in water) was prepared and placed in an autoclave. A magnetic stir bar was placed in the bottle before autoclaving.

After removal from the autoclave, the hot hydrogel was placed on a magnetic stirrer and, after cooling to about 80-90 °C while stirring all the time, hydroxyethylcellulose powder (medium viscosity, molecular weight of 170,000, viscosity of 1500-2500 cps) was added. The hydrogel was left on the stirrer to cool completely.

The prepared hydrogel was divided 200 mL each into sterile flasks.

The hydrogel from one flask was aliquoted as a control preparation, hydrogel alone, 5 mL each into sterile tube syringes (25 units) and 1 unit of about 8 mL for quality control (hereinafter QC).

*S. uberis* or *S. aureus* antigens were added to the other two in an amount equivalent to 40 doses (200 mL corresponds to 40 doses of 5 mL each) that is, 2×10¹¹ CFU.

The gel with antigens was also aliquoted, each into 25 tube syringes of 5 mL and into 1 QC of about 8 mL.

Sterility testing cultures were run from each last tube syringe (control), 1 mL each for EB and TSB. The cultures were incubated for 14 days. The sterility of the preparations was read: STERILE.

### 3.3 Preparation of preparations for animal immunization (vaccine, 2nd round)

The objective of the experiments was to prepare doses of the preparation for administration to the animals, in-calf heifers, for immunization in the 2nd round of the study. It was planned to administer 3 doses of the preparation about 4 and 2 weeks before calving and immediately after calving. The preparation is to be administered to all four quarters of the udder (4 teats) in a volume of 5 mL, each dose will contain 2×10⁹ CFU of inactivated bacteria. In the 2nd round, a preparation against *S. aureus* will be tested, containing 0.4×10⁸ CFU/mL (2×10⁹ CFU in 5 mL) of 4.2 LG₂ strain. The animals will be infected with *S. aureus* 16 MK₁ strain with the same genetic profile as the strain with which the animals are immunized.

Sterility testing culture of *S. aureus* 4.2 LG₂ strain antigen was run to confirm sterility. Tube syringes were assembled in a laminar flow cabinet. 1.5 L of 1% HEC hydrogel (1.35 L of water and 150 mL of glycerol) was prepared and autoclaved in a bottle with a magnetic stir bar. After the sterilization process, the bottle was set on the stirrer in the laminar flow cabinet and hydroxyethylcellulose (HEC) was gradually added until a 1% concentration was obtained; the preparation remained on the stirrer until it cooled completely.

The hydrogel was divided into two portions of 750 mL each. One portion (750 mL) was aliquoted as a preparation for the control group at 5 mL each into sterile tube syringes. To the second portion (750 mL), *S. aureus* antigen was added to obtain 0.4×10⁸ CFU/mL and aliquoted into tube tubes 5 mL each (obtaining a dose of 2×10⁹ CFU per teat, 8×10⁹ CFU per udder). The tube syringes were labeled and from the last one sterility testing cultures were run for EB (enriched broth) and TSB (tryptic soy broth) for quality control.

Results for sterility testing cultures were read: preparations were STERILE.

### Example 4

### Summary of the results of the of vaccine studies (2nd round) for S. aureus

The objective of the experiments was to determine the effect of an intramammary infusion (vaccine) in the form of antigen suspended in a hydrogel. The preparation was administered in 3 doses, approximately 4 and 2 weeks before calving and immediately after calving to all four quarters of the udder (4 teats) in a volume of 5 mL. Each dose contained 2×10⁹ CFU of inactivated bacteria. The preparation against *S. aureus,* contained 0.4×10⁸ CFU/mL (2×10⁹ CFU in 5 mL) of 4.2 LG₂ strain. The animals were infected with the *S. aureus* 16 MK₁ strain with the same genetic profile as the strain with which the animals were immunized.

The following groups of cows were formed for the study:
1. The group vaccinated and infected (in the left quarters) with *S. aureus:* 8 cows
2. The group infected in the left quarters, which was given pure hydrogel.

Milk was collected on days 0-7, 14, 21, 28, 35. IgG and IgM antibodies were tested from blood collected on days 0, 1, 7 and 14, 21, 28, 35. Cytokines from blood and milk on days 0-5 and 7.

The titers of antibodies (IgA, IgM, IgG) and cytokines (IL-1β, IL-8, TNF-a) in the milk and/or serum of cows treated from the fourth round of mastitis testing were also examined.

Milk and/or serum samples from cows were collected on day 0 (for both antibodies and cytokines), and on day 1-7 for cytokines and on day 1, 7, 14, 21, 28 and 35 for antibodies. Milk samples were collected from 4 udders (quarters). IgA levels were tested in milk from all quarters, IgG and IgM levels were tested from serum only (1 sample per cow), and cytokine levels were tested from both milk (2 quarters) and serum (1 sample per cow).

### RESULTS

### 3.1. Milk cultures + somatic cell counts

### Group 1

| **Cow #** | **Description of the results** |
|---|---|
| 0091 | SCC elevated until the last day of the experiment, noticeable upward tendency of the amount of bacteria in milk: from day 7 above the acceptable normal range |
| 1502 | Visible decreasing trend of SCC: from day 7 onward within the normal range. The amount of bacteria quite high during the whole experiment period. From day 14 an increase above the acceptable limit |
| 3938 | SCC in the left quarters high, practically until the end of the experiment above the normal range. The amount of bacteria during the whole experiment period below the acceptable limit |
| 4228 | SCC mostly low, on days 1.5; 4 and 28 in the left quarters SCC very high; the amount of bacteria almost during the whole experiment period remains below the acceptable limit. From day 28 in normal range |
| 3952 | SCC in the left quarters increases suddenly, amount of bacteria above the normal range on days 7 and 28 |
| 4307 | SCC in the left quarters high. Visible decrease from day 7, however, all the time above the normal range, similarly the number of bacteria. |
| 1491 | SCC in the left quarters very high until the end of the experiment. The amount of bacteria slightly above the normal range. |
| 1467 | Very high level of SCC, within normal range from day 7, the amount of bacteria changes during the whole experiment period, by day 28 at least one quarter shows infection above the acceptable limit |

### Group 2

| **Cow #** | **Description of the results** |
|---|---|
| 2349 | SCC in the left quarters high until day 7, then below the normal range. The amount of bacteria above the acceptable limit. From day 21 below the acceptable limit |
| 1481 | SCC in the left quarters high. So was the amount of bacteria. From day 14 the experiment ended-the cow died |
| 2877 | SCC high in the right quarters, very high amount of bacteria until the end of the experiment. |

| | |
|---|---|
| SCC - somatic cell count | |

### 3.2 ELISA results (cytokines, IgG, IgM and IgA antibodies)

### A. Cytokines

### a) TNF-a in milk

Tumor necrosis factor is known to be one of the main cytokines involved in the inflammatory and immune response, both in the acute and chronic phases of these processes. The main stimulus causing an increase in TNF-a synthesis by macrophages is lipopolysaccharides of the bacterial cell wall. Tumor necrosis factor, along with other cytokines, affects all cells of the immune system. TNF-a enhances the cytotoxicity of monocytes and macrophages, increases the phagocytic properties of neutrophils, and activates the cytotoxicity of eosinophils. TNF-a exhibits antiviral activity by inducing INF-γ-related intracellular defense processes, as well as antiparasitic activity through macrophage activation.

In the study groups, there are no significant differences in cytokine concentrations between the right and left quarters, and these concentrations remain at similar levels throughout the duration of the whole study. **This means that there was no local inflammatory reaction during the whole study period in both the vaccinated and control groups.**

### b) TNF-a in serum

TNF-a levels in each group were already high from day 0, however, they decreased after day 1 of the study and remained at about the same level until the end of the study, with a slight increase on day 7.

### c) IL-1β in milk

Cytokines of the interleukin-1 family play a key role in the inflammatory process and are secreted in response to various antigens of viral, bacterial and fungal origin. They also influence the processes of the adaptive immune response, inducing the secretion of interleukin-6 by T cells and affecting the development of B cells. IL-1β is responsible for most of the effects induced by IL-1. An increase in the concentration of this mediator is the direct cause of prostaglandin E2 (PGE2) synthesis in the hypothalamus, and thus an increase in body temperature (fever). Interleukin-1β is also responsible for the activation of neutrophils and endothelial cells, the accumulation of neutrophils in the circulatory system and the production of antibodies by lymphocytes.

### d) IL-1β in serum

In the case of interleukin-1β, no strong correlation between groups can be seen. Some similarities can be seen between the quarters, the biggest differences are between the quarters of cows given hydrogel. **Low comparable levels of interleukin in each group indicate a low local, inflammatory response to bacterial antigens (despite the performed infection).**

In the case of serum IL-1β concentrations, a very large increase is evident in the hydrogel-administered group. Almost two times higher concentration was maintained during the whole period of the experiment. **The results obtained indicate that the body was protected from inflammation after vaccination.**

### e) IL-8 in milk

Interleukin-8 (IL-8) is a cytokine that stimulates the migration of immune cells in the body. This means that it stimulates the movement and proliferation of T cells, neutrophils and monocytes. This action is defensive in its nature. IL-8 stimulates the release of histamine by basophils. This process triggers an allergic reaction.

In the case of IL-8, similarities can be observed between quarters within groups. The highest concentrations of the cytokine were recorded in vaccinated and infected cows -> **there was an increased stimulation of the immune system in the vaccinated group.** The control (hydrogel) group presented fairly low levels of interleukin during the whole experiment period.

### e) IL-8 in serum

IL-8 in serum showed significantly elevated levels in the vaccinated group, while it remained quite low during the whole experiment period in the group to which the hydrogel was administered. **This confirms the results obtained for interleukin content in milk.**

### B. Antibodies

### a) IgA in milk

Two types of immunoglobulin class A are known: serum (slgA) and secretory. IgA antibodies are the predominant class of immunoglobulins in human mucosal and serous (e.g., nasopharyngeal or bronchial) secretions, in which they are found mainly in dimeric form. In serum, IgA antibodies are found primarily in monomeric form. Class IgA antibodies are considered the body's first line of defense against harmful external environmental agents. slgA antibodies are responsible i.a. for neutralizing viruses, toxins and enzymes produced by microorganisms. In serum, IgA antibodies play a complementary role, and are involved, *i.a*., in neutralizing those antigens that have crossed the mucosal barrier and entered the bloodstream. Synthesis of IgA class antibodies is induced mainly in mucous membranes, but also systemically.

IgA antibodies showed a significant increase in the left udder quarters of vaccinated cows (mainly on days 7-14). Cows given hydrogel showed very high levels of antibodies on day 0, after which a significant increase was observed. **These results confirm previous studies about immune system stimulation in vaccinated cows.**

### b) IgG in serum

IgG antibodies are produced in the next phase of the infection, roughly from about 14 days after disease onset, and over time their number increases until it reaches a certain level. IgG antibodies remain in the body for a long time, sometimes even for a lifetime, to activate almost immediately upon re-exposure to a particular type of pathogen and quickly fight it off (in the event of a subsequent infection, the amount of IgG increases rapidly). Elevated IgG levels are also observed due to ongoing infectious, inflammatory processes.

All groups had a similar course of the experiment, an increase was observed on day 21 (hydrogel group presented slightly higher levels). On day 35, both groups showed the same amount of IgG antibodies (level close to the initial one). **The obtained results showed that the vaccine protected the body from pathogens, so there was less activation of antibodies.**

### c) IgM in serum

IgM antibodies appear in the early stages of infection. They are produced as the first ones, and their presence is indicative of a developing disease; over time, their amount decreases and the amount of IgG antibodies increases. These have the task of eliminating pathogens before the body produces sufficient amounts of further antibodies, as well as activating proteins present in the plasma.

The amount of IgM antibodies increased significantly in the vaccinated group on days 21-28. In the control group, it remained at a fairly low level throughout the experiment. On day 35 the level was similar in both groups.

### 3.3. Conclusions of the study

- The lack of a significant increase in TNF indicates a limited inflammatory response in the study groups.
- IgA antibodies are considered the body's first line of defense against harmful external environmental factors and are responsible i.a. for neutralizing viruses, toxins and enzymes produced by microorganisms. IgA antibodies showed a significant increase in the left udder quarters of vaccinated cows (mainly on days 7-14). Cows given hydrogel showed very high levels of antibodies on day 0, after which a significant increase was observed. **These results confirm previous studies about immune system stimulation in vaccinated cows.**
- The amount of IgM antibodies increased significantly in the vaccinated group on days 21-28. In the control group, it remained fairly low during the whole period of the experiment. On day 35, the level was similar in both groups.
- The level of IgG antibodies is noticeable in the next phase of infection, roughly from about 14 days after the disease onset, and over time their number increases until it reaches a certain level.

All groups had a similar course of the experiment, the increase was observed on day 21 (hydrogel group presented slightly higher level). On day 35, both groups showed the same amount of IgG antibodies (level close to the initial one). **The obtained results showed that the vaccine protects the body from pathogens, so there was less activation of antibodies.**
- Both the vaccinated and control (hydrogel administration) groups had elevated levels of SCC in the left quarters throughout almost the entire period of the experiment. This was probably due to the performed infection. The vaccine allowed maintaining the amount of bacteria below the acceptable limit in most cases. One cow died in the control group. The above results suggest that the vaccine has a protective effect against the development of mastitis.

### Example 5

### Safety and efficacy studies of the preparations

In order to test the safety and efficacy of the preparations, milk and blood samples were collected from cows for analysis. Testing the levels of both cytokines and antibodies from milk is one of the tests that provide information about the changes occurring directly in the mammary gland caused by inflammation, while testing the levels of cytokines and antibodies from blood is able to show us what changes are occurring on the scale of the whole organism. During tests, specific antibody titers (IgA, IgM, IgG) were determined to assess the type of immune response and cytokines (IL-1β, IL-8, TNF-a) in the milk of cows. The levels of specific antibodies to *S. aureus* and *S. uberis* antigens were tested. Samples from cows were collected on day 0 (for both antibodies and cytokines), and on day 1, 2, 3 and 4 for cytokines, and on day 7, 14, 21, 28 and 35 for antibodies. Samples were collected from 4 udders (quarters). Cows were administered hydrogel with the appropriate antigens or without antigen, for control. In addition, shedding tests, animal temperature measurements and SCC measurements were carried out.

Safety studies of the vaccine were conducted first. Once this assumption was confirmed, the vaccines were tested against pathogens:

### S. UBERIS

- The lack of a significant increase in TNF indicates a limited inflammatory response in the study groups.
- IgA antibodies are considered the body's first line of defense against harmful external environmental factors and are responsible *i.a.* for neutralizing viruses, toxins and enzymes produced by microorganisms. IgA antibodies showed a significant increase in the left udder quarters of vaccinated cows (mainly on days 7-14). Cows given hydrogel showed very high levels of antibodies on day 0, after which a significant increase was observed. **These results confirm previous studies about immune system stimulation in vaccinated cows.**
- The amount of IgM antibodies significantly increased on days 21-28 in the vaccinated group. In the control group, it remained fairly low during the whole period of the experiment. On day 35, the level was similar in both groups.
- The level of IgG antibodies is noticeable in the next phase of infection, roughly from about 14 days after the disease onset, and over time their number increases until it reaches a certain level.

All groups had a similar course of the experiment, the increase was observed on day 21 (hydrogel group presented slightly higher level). On day 35, both groups showed the same amount of IgG antibodies (level close to the initial one). **The obtained results showed that the vaccine protected the body from pathogens, so there was less activation of antibodies.**
- Both the vaccinated and control (hydrogel administration) groups had elevated levels of SCC in the left quarters during almost the whole period of the experiment. This was probably due to the infection performed. The vaccine allowed maintaining the amount of bacteria below the acceptable limit in most cases. The above results suggest that the vaccine has a protective effect against the development of mastitis.

**CONCLUSION** *S. uberis:* The SCC was elevated in most of the cows tested, the amount of bacteria in milk remained at the limit of the normal range.

### S. AUREUS

- The vaccine allowed maintaining the amount of bacteria below the acceptable limit in most cases.
- The lack of a significant increase in TNF indicates a limited inflammatory response in the study groups.
- IgA antibodies are considered the body's first line of defense against harmful external environmental factors and are responsible *i.a.* for neutralizing viruses, toxins and enzymes produced by microorganisms. IgA antibodies showed a significant increase in the left udder quarters of vaccinated cows (mainly on days 7-14). Cows given hydrogel showed very high levels of antibodies on day 0, after which a significant increase was observed. These results confirm previous studies about stimulation of the immune system in vaccinated cows.
- The amount of IgM antibodies significantly increased in the vaccinated group on days 21-28.

In the control group, it remained at a fairly low level during the whole period of the experiment.
- The level of IgG antibodies is noticeable in the next phase of infection, roughly from about 14 days after the disease onset, and over time their number increases until it reaches a certain level.

All groups had a similar course of the experiment, an increase was observed on day 21 (hydrogel group presented slightly higher levels). On day 35, both groups showed the same amount of IgG antibodies (level close to the initial one). The obtained results showed that the vaccine protected the organism from pathogens, so there was less activation of antibodies.

**CONCLUSION** *S. aureus:* Both SCC and bacterial levels in milk were elevated during the whole period of the experiment in most cows, however at much lower levels than for the control group.

In the vaccine safety experiment (Exp. 1; n=18), placebo (NaCl, n=6) or vaccine (n=12) were administered by intramammary infusion. It was shown that there were no side effects of the intramammary use of the developed vaccine. The vaccine did not adversely affect feed intake, health parameters and udder condition.

In the proper experiment (n=32, immunization against pathogens), the preventive efficacy of the vaccine was demonstrated. The efficacy of intramammary vaccination against experimentally induced udder inflammation (intramammary infusions of pathogens: *Staphylococcus aureus* and *Streptococcus uberis*)*.* The animals were vaccinated as in Exp. 1. Milk samples and blood from the external jugular vein were collected twice daily on days: -1, 0 (infection), 1, 2, 3, 4, 5, 7, 14, 21, 28, 35, and blood from the milk vein once daily. On day 35, udder tissue was collected. Animal health was monitored and the following analyses were performed:
**Milk analysis:** High somatic cell count and LDH levels in milk persisted until day 7 after infection and differed on day 35 in control and vaccinated groups.

**Complete blood count:** Changes in hematological parameters were observed in cows with severe mastitis without vaccination (no immunization) compared to the vaccinated animals.

**Blood biochemistry:** A Health Check Panel was used which included: ALB, TP, GLOB, TB, AST, ALT, AMY, CK, CREA, BUN, GLU, TG, Ca, PHOS. Changes in parameters (mainly CK and CREA) were observed in cows with severe mastitis without vaccination compared to vaccinated animals.

**ELISA:** Levels of TGF-β, PGE2 and PGF2a and mediators of extracellular matrix remodeling (MMP-9 and TIMP-1) differ in vaccinated cows compared to controls. A decrease in the concentrations of the studied mediators was observed along with the healing process.

**Gene expression:** In mammary gland tissue, changes in the expression of the studied factors: *Bax, BCL-2, CASP3, CASP8, RIPK1* and *RIPK3, ELA, CAT, MPO, collagen I, III, IV, fibronectin, hyaluronic acid, MMP-9* at the gene level differed in the vaccinated cows compared to controls.

Parameters (SCC, LDH) in milk samples of infected cows after vaccination were lower compared to controls. In calves, there were no differences in weight gain and health parameters among the study groups. The vaccine administration did not contribute to early parturitions or miscarriages.

Mechanisms of action and efficacy of antigen-containing infusions (vaccine) have been demonstrated.

The absence of local mastitis symptoms: swelling, redness, soreness of the udder, body temperature of 38.0±0.5 was observed. The preparation is effective for *S. aureus* and *S. uberis:* the results of the study groups' milk parameters include lower amounts of SCC and LDH (significance level p<0.05) compared to the control group.

The study showed a potential protective effect on heifers against the onset of severe mastitis symptoms. The increase in the somatic cell count in the first days of the study was probably due to a breach in the physiological barrier of the teat. The amount of bacteria in the milk, in most cases, was within acceptable ranges.

The efficacy of inducing immunity after administration of a intramammary infusion containing antigens (the level of specific IgG or IgM and/or secretory IgA, respectively, in serum and milk samples of cows from the study groups higher than the level of these antibodies in the milk of cows from the control group (at the significance level of p<0.05)) was demonstrated.

**OBSERVATIONS:** Based on the obtained results, it was concluded that the vaccine has a protective effect against the development of mastitis. However, further studies involving different doses or vaccination regimens are needed to accurately determine the extent of the vaccine efficacy and optimal conditions of use. Regardless of the promising results of the first studies, there are many factors that affect the efficacy of vaccines, such as the diversity of bacterial strains, culturing conditions or the individual immune response of animals, etc. Therefore, further studies are needed to better understand vaccine efficacy under different conditions.

### Example 6

### Analysis of beneficial effects compared to other preparations

### Description of other preparations:

A competing product to the intramammary infusion containing inactivated antigens of mastitis-causing bacteria is the vaccine. Among the available on the European market preparations with an immunizing effect against udder inflammation are:
- Mastibiovac/BIOWET DRWALEW/
- Startvac/HIPRA/
- Ubac/HIPRA/.

These preparations are administered to cows by injection (intramuscularly or subcutaneously). In the case of mastitis the literature shows that this route of administration produces little or no protective effect [Freick et *al.,* 2016; Landin et *al.,* 2015]. Intramammary administration of the vaccine will stimulate antibody production directly in the organ [Bharathan, Mullarky, 2011].

The developed immunological preparation for the prophylaxis of udder inflammation (mastitis) in cows is for intramammary administration, which is a novel solution. Competing preparations on the market are for subcutaneous or intramuscular administration.

### Example 7

### Brief description of vaccine production

Inoculate LB medium with bacterial overnight culture. Maintain the culture at 37 °C with aeration for 24 h. Optionally, an antifoaming agent can be added. Inactivate the culture with 10% formalin to obtain a concentration of 0.1% and incubate further at 37 °C with agitation without addition of air for min. 3h. Then transfer the culture to a sterile vessel, seal tightly and inactivate further overnight under the same conditions. Centrifuge the inactivated bacteria and suspend the pellet in 0.85% NaCl with 0.02% formalin. Prepare a hydrogel (10% glycerol in water) and place in an autoclave. After removing from the autoclave, put the hot hydrogel on a magnetic stirrer and after cooling to about 80-90 °C with constant stirring, add hydroxyethylcellulose powder of medium viscosity (molecular weight of 170,000, viscosity of 1500-2500 cps), until a 1% concentration is obtained. Leave the hydrogel on the stirrer to cool completely. Add *S. uberis* or *S. aureus* antigens to the hydrogel in the appropriate amount and stir. Aliquote the gel with antigens into injection bottles.

### Summary

The proposed vaccine is an effective and innovative strategy for the prophylaxis and/or treatment of udder inflammation in cows.

Successful treatment of mastitis in cows requires an individualized approach taking into account a number of factors, such as the type of pathogen, the stage of the disease, the overall health condition of the animal and specifics of the farming. If mastitis is suspected in cows, it is always advisable to consult a veterinarian, who can conduct appropriate tests and plan appropriate therapy.

## Claims

1. A vaccine for use in the prevention and/or treatment of udder inflammation (mastitis) in cows, **characterized in that** it contains preparation a) and/or preparation b), wherein
preparation a) contains as active substance *Staphylococcus aureus* 4.2 LG₂ strain, inactivated with formaldehyde for intramammary administration, with the following characteristics:
| Feature/strain | 4.2 LG₂ |
|---|---|
| Identification to species | nuc |
| Toxicity genes | - |
| Genes encoding surface proteins | *IsdC, EsxA, IsdA, SdrD, IsdB, Bap,* and *SdrC* |
| Antibiotic resistance | to most antibiotics tested, including cloxacillin, cefoperazone, cefquinome, etracycline, neomycin, kanamycin, and gentamicin |
| Sensitivity | amoxicillin plus clavulanic acid, penicillin, cephalexin, streptomycin, and bacitracin |
biochemical diagnostics (ErbaScan) for *S. aureus* 4.2 LG strain
| URE | ARG | ORN | BGA | GLR | BGL | PHS | ESL | NAG | GAL | SUC | TRE | MAN | MLT | XYL | MNS |
|---|---|---|---|---|---|---|---|---|---|---|---|---|---|---|---|
| + | + | - | - | - | + | + | + | + | + | + | + | + | + | - | + |
| LAC | SOR | RIB | FRU | CEL | ARA | RAF | XOL |
|---|---|---|---|---|---|---|---|
| + | - | + | + | + | - | - | - |
preparation b) contains as an active ingredient *S. uberis* 19MK and 7MO strains, inactivated with formaldehyde, for intramammary administration, with the following characteristics:
| | |
|---|---|
| Feature/strain | 19MK |
| Identification to species | *165 rRNA* |
| Toxicity genes | *cfu* |
| Genes encoding surface proteins | *gapC* |
| Antibiotic resistance | to all antibiotics tested |
biochemical diagnostics (ErbaScan) for 19MK
| | | | | | | | | | | | | | | | |
|---|---|---|---|---|---|---|---|---|---|---|---|---|---|---|---|
| HIP | PHS | LAP | GLR | AGA | ESL | ARG | URE | MAN | SOR | TRE | LAC | RAF | INU | MLB | RIB |
| + | - | + | - | - | + | + | - | + | + | + | + | - | - | - | + |
| | |
|---|---|
| Feature/strain | 7MO |
| Identification to species | *165 rRNA* |
| Toxicity genes | *sua* |
| Genes encoding surface proteins | *skc, pauA, gapC, oppF, hasA, hasB, hasC* |
| Antibiotic resistance | resistant to most antibiotics tested |
| Sensitivity | shows sensitivity or medium sensitivity to penicillin, cephalexin, erythromycin, and bacitracin |
biochemical diagnostics (ErbaScan) for 7MO
| HIP | PHS | LAP | GLR | AGA | ESL | ARG | URE | MAN | SOR | TRE | LAC | RAF | INU | MLB | RIB |
|---|---|---|---|---|---|---|---|---|---|---|---|---|---|---|---|
| + | - | + | + | - | + | + | - | + | + | + | + | - | - | - | + |

2. The vaccine for use according to claim 1, **characterized in that** the preparation a) contains 0.4×10⁸ CFU/mL (2×10⁹ CFU in 5 mL) of a formaldehyde-inactivated *S. aureus* strain.

3. The vaccine for use according to claims 1 or 2, **characterized in that** the preparation b) contains 0.2×10⁸ CFU/mL of *S. uberis* 19MK strain and 0.2×10⁸ CFU/mL of *S. uberis* 7MO strain inactivated with formaldehyde.

4. The vaccine for use according to claims 1 or 2 or 3, **characterized in that** it additionally contains preparation c) containing further inactivated bacterial strains, preferably *Escherichia coli, Klebsiella pneumoniae* and *Klebsiella oxytoca* and *Streptococcus dysgalactiae.*

5. The vaccine for use according to any of claims 1-4, **characterized in that** it contains saline, hydrogel (10% glycerol in water, 1% hydroxyethylcellulose powder with a viscosity of 1500 to 2500 cps), formaldehyde as additional substances in all preparations.

6. The vaccine for use according to any of claims 1-5, **characterized in that** it is administered according to the following scheme:
| **Administration** | **Age of the animal** | **Composition of the intramammary dose** |
|---|---|---|
| I dose to each teat in volume of 5 mL | heifers min. 20 months old, about 4 weeks before calving | 0.4×10⁸ CFU/mL (2×10⁹ CFU in 5 mL) of inactivated *S. aureus* 4.2 LG₂ strain bacteria or inactivated *S.* uberis bacteria, containing 0.2×10⁸ CFU/mL of 19MK strain and 7MO strain (mixed 1:1, total of 0.4×10⁸ CFU/mL → 2×10⁹ CFU in 5 mL) and suspended in 1% hydrogel. |
| II dose to each teat in volume of 5 mL | heifers min. 20 months old, about 2 weeks before calving | 0.4×10⁸ CFU/mL (2×10⁹ CFU in 5 mL) of inactivated *S. aureus* 4.2 LG₂ strain bacteria or inactivated *S.* uberis bacteria, containing 0.2×10⁸ CFU/mL of 19MK strain and 7MO strain (mixed 1:1, total of 0.4×10⁸ CFU/mL → 2×10⁹ CFU in 5 mL) and suspended in 1% hydrogel. |
or
| **Administration** | **Age of the animal** | **Composition of the intramammary dose** |
|---|---|---|
| I dose to each teat in volume of 5 mL | heifers min. 20 months old, about 4 weeks before calving | 0.4×10⁸ CFU/mL (2×10⁹ CFU in 5 mL) of inactivated *S. aureus* 4.2 LG₂ strain bacteria and suspended in 1% hydrogel. |
| II dose to each teat in volume of 5 mL | heifers min. 20 months old, about 2 weeks before calving | 0.4×10⁸ CFU/mL (2×10⁹ CFU in 5 mL) of inactivated *S. aureus* 4.2 LG₂ strain bacteria and suspended in 1% hydrogel. |
| III dose immediately after calving, to each teat in volume of 5 mL | heifers min. 20 months old, on the day of parturition | 0.4×10⁸ CFU/mL (2×10⁹ CFU in 5 mL) of inactivated *S. aureus* 4.2 LG₂ strain bacteria and suspended in 1% hydrogel. |

7. A method for obtaining a vaccine as defined in any of claims 1-6, **characterized in that** it includes steps in which:
- a culture containing at least one strain selected from such as *Staphylococcus aureus, Streptococcus uberis, Escherichia coli, Klebsiella pneumoniae* and *Klebsiella oxytoca* and *Streptococcus dysgalactiae* is inactivated with 10% formalin to obtain a concentration of 0.1% and incubated at 37 °C with stirring without air for at least 3h;
- the culture then is transferred to a sterile vessel and inactivated while tightly sealed overnight under the same conditions;
- the inactivated bacteria are centrifuged and the pellet is suspended in 0.85% NaCl solution with 0.02% formalin;
- a hydrogel containing 10% glycerol in water is prepared, heated to at least 95 °C, then after cooling to about 80-90 °C with stirring, hydroxyethylcellulose with a viscosity of 1500 to 2500 cps in powder form is added to 1% concentration and the hydrogel is stirred until completely cooled;
- the suspension of inactivated bacterial strains obtained above is added to the resulting hydrogel and stirred.

8. A use of the vaccine defined in claims 1-5 for the manufacture of an immunizing and/or therapeutic preparation against udder inflammation (mastitis) in cows.
